# EUROPEAN PATENT APPLICATION

(11) **EP 4 159 274 A1**
(43) Date of publication of application: **05.04.2023**
(21) Application number: 21813427.8
(22) Date of filing: 25.03.2021
(51) Int. Cl.: A61N 1/39, A61N 1/04, B05C 11/10, B05C 11/11, F16J 15/10

(54) **ELECTRODE PLATE AND WEARABLE DEFIBRILLATION DEVICE**

(30) Priority: 29.05.2020 CN 202010478035
(71) Applicant: MicroPort Soaring CRM (Shanghai) Co., Ltd., Shanghai 201203 (CN)
(72) Inventor: HE, Mingchen, Shanghai 201203 (CN)
(74) Representative: Lavoix
(86) International application number: PCT/CN2021/083107
(87) International publication number: WO 2021/238375

(57) **Abstract**

An electrode plate (100) and a wearable defibrillation device are disclosed. The electrode plate (100) includes a hermetic shell (110) and a capsule (120) disposed in the hermetic shell (110). The hermetic shell (110) has an inflation port (111) and an overflow aperture (112). The overflow aperture (112) is provided in an exposed surface (113) of the hermetic shell (110), which has electrical conductivity. The capsule (120) includes a body (121) and a cover (124). The body (121) defines a hollow cavity (122) and an outlet orifice (123) in communication with the cavity (122). The cavity (122) is configured for storage of a conductive paste (S) therein and is isolated from a hollow internal space of the hermetic shell (110). The cover (124) is disposed over the outlet orifice (123) so as to close the outlet orifice (123). When the cover (124) is broken open, an opening therein resulting from the breakage comes into communication with the outlet orifice (123) and the overflow aperture (112), allowing the conductive paste (S) in the cavity (122) to flow successively through the outlet orifice (123), the opening in the cover (124) and the overflow aperture (112) onto the exposed surface (113). Such automatic application of the conductive paste (S) can provide timely protection to a patient and enhance safety in release of the conductive paste (S).

## Description

### TECHNICAL FIELD

The present invention relates to the field of medical devices and, in particular, to an electrode plate and a wearable defibrillation device.

### BACKGROUND

In life, cardiac ventricular fibrillation is a commonly seen heart disease, it is characterized by no sign of onset, a short rescue time (only 4 minutes from the onset to death) and a high sudden death rate. For patients with this disease, the current emergency treatment approach is mainly to defibrillate the heart by delivering a high DC voltage to the heart, restoring the heart to a normal rhythm. Existing defibrillator devices can be categorized primarily into the following four types:
(1) mobile defibrillators, which can be moved but are inconvenient to carry due to bulkiness and therefore mostly deployed in hospitals;
(2) automated external defibrillators (AED), which are easy to carry and usually placed at highly noticeable locations in public places;
(3) implantable cardioverter-defibrillators (ICD), which are operable in a fully automated manner and can be implanted into patients; and
(4) wearable cardioverter-defibrillators (WCD), which are operable in a fully automated manner and used principally for patient protection from diagnosis to ICD implantation and on patients unsuitable for ICD implantation.

The former two types of defibrillator devices require manual operation. However, a patient usually loses consciousness within ten to twenty seconds after onset, and such a short period of time would present extreme challenges to manual operation. Therefore, these devices are not suitable for constant patient protection. By contrast, the latter two types of defibrillator devices are both operable in a fully automated manner not requiring manual operation and thus suitable for constant patient protection. At present, WCD devices have been widely used in clinical practice because they do not require surgical implantation and can be easily removed.

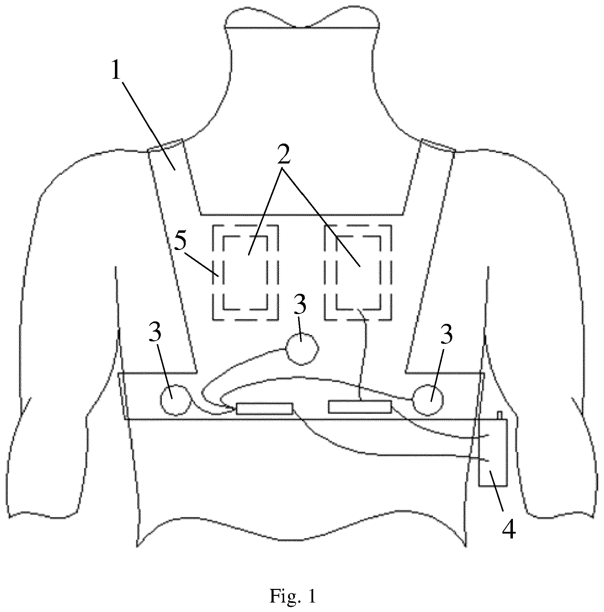

Fig. 1 explains how a conventional wearable cardioverter-defibrillator (WCD) device is used. As shown in Fig. 1, the WCD device is meant to be worn on the upper torso in the form of a shoulder strap harness and includes a shoulder strap 1, a defibrillation pad 2, a sensing electrode 3, a host 4 and an airbag 5. The specific operating principle is that the sensing electrode 3 senses an ECG signal and feeds the ECG signal to the host 4. The host 4 responsively produces an electrocardiogram and makes a determination based on the electrocardiogram. If it is determined that the patient is experiencing ventricular fibrillation, then the airbag 5 on the shoulder strap 1 is inflated to compress the defibrillation pad 2 against the patient's skin, and the defibrillation pad 2 is caused to deliver a high DC voltage for defibrillation treatment. One challenge with this defibrillator device is that, before the electric defibrillation by the electrode plate, the contact resistance between the defibrillation pad 2 and the skin must be small enough, otherwise a relatively large contact resistance would tend to burn the patient's skin and myocardium when the electric defibrillation by the defibrillation pad, or even fail defibrillation for emergency protection of the patient.

In order to tackle this challenge, a capsule containing a conductive paste is burst under the action of a large amount of gas produced by an ignited gas-producing pellet, so that the defibrillation pad is coated, thereby reducing impedance between the conductive surface of the therapy electrode and the patient's skin. However, this approach is disadvantageous in that, in order to create a gas pressure by explosion of the gas-producing agent, which is sufficiently high to cause the gas to break open the capsule, a box for enclosing the capsule must be designed to be as strong as possible. As a result, the box has to have sufficiently thick and hard walls, which would cause discomfort to a patient who is wearing the device and make him/her not willing to put it on anymore. Moreover, it is difficult to guarantee the safety of the gas-producing agent. Further, in case multiple capsules are used, if there are strength differences between them, individual capsules may be broken open. As the broken ones will cause gas leakage and hence an abrupt pressure drop, the others cannot be broken any more and the conductive paste contained therein cannot be released. As a consistent amount of released conductive paste cannot be guaranteed, it is impossible to ensure that the defibrillator device can always function in a timely way to provide the patient with emergency protection. Thus, the approach suffers from insufficient reliability.

### SUMMARY OF THE INVENTION

In order to overcome the above-described problems, it is an object of the present invention to provide an electrode plate and a wearable defibrillation device, which enables automatic coating of a conductive paste and can protect a patient in a timely manner. Moreover, the conductive paste is released in a reliable and safe manner. In particular, the electrode plate can be made lightweight and slim enough to increase the patient's wearing comfort and compliance.

To this end, in one aspect of the present invention, there is provided an electrode plate for use in cardiac defibrillation, which includes a hermetic shell and a capsule disposed in the hermetic shell. The hermetic shell has an inflation port and an overflow aperture. The overflow aperture is provided in an exposed surface of the hermetic shell, which is conductive.

The capsule includes a body and a cover. The body defines a hollow cavity and an outlet orifice in communication with the cavity. The cavity is configured for storage of a conductive paste therein and is isolated from a hollow internal space of the hermetic shell. The cover is disposed over the outlet orifice so as to close the outlet orifice. When the cover is broken open, an opening therein resulting from the breakage will come into communication with the outlet orifice and the overflow aperture.

Optionally, the cover may be configured to, when a gas is introduced into the hermetic shell, be broken open by heating and/or under the action of a pressure of the gas.

Optionally, the electrode plate may further include a heating element attached to the cover on the side thereof opposite to the outlet orifice, wherein the cover is configured to be heated, molten and broken open when the heating element is energized.

Optionally, the cover may be configured with a weakened feature which is able to withstand a maximum pressure lower than a maximum pressure that the rest of the cover is able to withstand.

Optionally, the electrode plate may further include a heating element disposed on the side thereof opposite to the outlet orifice, the heating element attached to the cover by an adhesive, at least part of which is applied to the weakened feature, and which is configured to be heated and molten when the heating element is energized, wherein the cover is configured to be heated, molten and broken open when the heating element is energized.

Optionally, the weakened feature may be configured as a non-through indentation.

Optionally, the body may define a mounting recess around the outlet orifice, wherein the cover is sheet-shaped and fits into the mounting recess of the body.

Optionally, the cover may be a single-sided adhesive film consisting of two layers, with the heating element being inserted between the two layers.

Optionally, two or more than ten of the capsules may be included.

Optionally, the electrode plate may include a plurality of the capsules, each of the capsules defining an elongate outlet orifice provided with a plurality of overflow apertures arranged in a row, each of the overflow apertures being elongate and having the same lengthwise direction as the outlet orifice.

Optionally, the hermetic shell may include a front shell half and a rear shell half, which are both insulators and coupled together to form the hermetic shell, the front shell half providing the exposed surface, the front shell half having material strength higher than material strength of the rear shell half, the rear shell half configured to expansively deform as a result of inflating the hermetic shell.

Optionally, the rear shell half may be provided, on the side thereof facing the front shell half, with a protrusion configured to abut against the capsule.

Optionally, the outlet orifice and the overflow aperture may be configured so that, after the cover is broken open, the conductive paste stored in the cavity flows out of the outlet orifice through the overflow aperture onto the exposed surface.

In another aspect of the present invention, there is provided a wearable defibrillation device including the electrode plate as defined in any of the preceding paragraphs.

In the electrode plate and the wearable defibrillation device provided in the present invention, the electrode plate includes the hermetic shell and the capsule disposed in the hermetic shell, the capsule is configured for storage of the conductive paste therein, and the hermetic shell can release the conductive paste through the overflow aperture. In practical use, an inflation pump included in a wearable defibrillation device is allowed to be used to introduce a gas into a hermetic shell via the inflation port to cause expansive deformation of the hermetic shell. In this process, when the cover on the capsule is broken open, under the action of a gas pressure within the hermetic shell, the conductive paste within the capsule can flow out of an opening in the cover resulting from the breakage through the overflow aperture onto the conductive exposed surface. In this way, apart from automatic application of the conductive paste which enables timely protection of a patient, higher safety can be obtained by avoiding the use of a gas-producing agent for creating a gas pressure rise by explosively producing a gas.

In addition, instead of a sufficiently high pressure resulting from an explosion of a gas-producing agent, the breakage of the cover on the capsule can be simply accomplished by introducing an appropriate amount of gas into the hermetic shell. In this process, the cover may break as a result of a pressure of the gas introduced into the hermetic shell, a heating action, or both. In these approaches for causing the breakage, the gas pressure can be controlled by the inflation pump included in the wearable defibrillation device, thus ensuring that the cover is broken at a maximum limit pressure lying within an acceptable safe pressure range for the human body and ensuring safety in release of the conductive paste. In particular, unlike a gas pressure produced by an instantaneous explosion of a gas-producing agent, the pressure of the gas filled in the hermetic shell is balanced, ensuring simultaneous breakage of all capsules and hence release of a sufficient amount of the conductive paste. As such, the defibrillator device is enabled to function in a timely manner to protect the patient in emergency situations. Further, heating the cover enables the conductive paste that is squeezed out to be also heated and thus have increased fluidity. In this way, release of the conductive paste is enhanced.

In particular, breaking the cover by a heating action and/or under the action of a gas pressure created by an inflation action enables the hermetic shell of the electrode plate to be designed sufficiently lightweight and slim to increase wearing comfort and compliance. Moreover, the electrode plate of the present invention is an inexpensive disposable product and can relieve the patient's treatment burden.

Further, in order to ensure breakage of the cover under the action of the gas pressure, the cover is preferably provided thereon with a weakened feature preferably in the form of a non-through indentation. In this case, when the cover is subjected to a thermal load and/or a pressure load, the weakened feature will be ruptured first. This is advantageous in structural simplicity, ease of operation and safe and reliable breakage of the cover. Additionally, in order to ensure that the capsule will not be accidentally damaged during daily wearing, an adhesive is preferably applied around the weakened feature to enhance the structural strength of the weakened feature. Under normal circumstances, the adhesive may be heated and molten by the heating element to ensure breakage of the cover.

Further, the hermetic shell in the electrode plate preferably includes the front shell half and the rear shell half. The material strength of the front shell half is higher than that of the rear shell half, and the rear shell half is configured to expansively deform as a result of inflating the hermetic shell. With this arrangement, on the one hand, the electrode plate is overall soft, additionally enhancing wearing comfort, and on the other hand, the electrode plate still exhibits sufficient toughness, which prevents defibrillation performance degradations of the electrode plate when it is twisted and deformed during the patient's daily wearing, for example, during his/her physical activities. Apart from these, the rear shell half of the electrode plate is preferably provided on the side thereof facing the front shell half with the protrusion, which can prevent the capsule from being accidentally crushed and broken during daily wearing.

Furthermore, multiple capsules may be included in the electrode plate to enable an increased amount of released conductive paste, a larger coated area and higher defibrillation safety. In particular, when a gas is filled into the hermetic shell, as the cavity of each capsule is isolated from the hollow internal space of the hermetic shell, and since all the capsules are subject to the same gas pressure conditions, even if the capsules fails to be simultaneously broken, one or more of them that have been broken prior to the other(s) will not lead to a pressure drop in the hermetic shell and thus will not make the remaining one(s) impossible to be broken any longer. Therefore, reliable release of the conductive paste can be ensured. Moreover, according to the present invention, the electrode plate may include two capsules and can accommodate a greater amount of the conductive paste at a given defibrillation area. In this way, an increased amount of the conductive paste can be released to achieve even safer defibrillation. In particular, the electrode plate can be made even more lightweight and slimmer, imparting even better wearing comfort to the patient.

### BRIEF DESCRIPTION OF THE DRAWINGS

The accompanying drawings are provided to facilitate a better understanding of the present invention and do not unduly limit the scope thereof in any sense. In these figures:
Fig. 1 explains how a conventional wearable cardioverter-defibrillator is used;
Fig. 2 is a schematic assembled view of an electrode plate according to a first preferred embodiment of the present invention;
Fig. 3 is a schematic exploded view of the electrode plate according to the first preferred embodiment of the present invention, in which front and rear shell halves are being coupled together;
Fig. 4 is a front view of a capsule according to the first preferred embodiment of the present invention, without an electrical heating wire being attached thereto;
Fig. 5 is a cross-sectional view of the capsule taken along line A-A in Fig. 4;
Fig. 6 is another schematic exploded view of the electrode plate according to the first preferred embodiment of the present invention, in which the front and rear shell halves are separated from each other;
Fig. 7 is a schematic assembled view of the electrode plate according to the first preferred embodiment of the present invention, in which a conductive panel is omitted;
Fig. 8 is a front view of the electrode plate according to the first preferred embodiment of the present invention;
Fig. 9 is a cross-sectional view of the electrode plate taken along line B-B in Fig. 8, in which the rear shell half is omitted;
Fig. 10 is a schematic front assembled view of the capsule according to the first preferred embodiment of the present invention, with an electrical heating wire being attached thereto;
Fig. 11 is a schematic exploded view of the capsule according to the first preferred embodiment of the present invention,
Fig. 12 is a schematic back assembled view of the capsule according to the first preferred embodiment of the present invention;
Fig. 13 explains how a wearable defibrillation device according to the first preferred embodiment of the present invention is used;
Fig. 14 is a schematic exploded view of an electrode plate according to a second preferred embodiment of the present invention;
Fig. 15 schematically illustrates a front shell half according to the second preferred embodiment of the present invention;
Fig. 16 is a schematic diagram showing an internal side of a front shell half attached thereto with capsules according to the second preferred embodiment of the present invention;
Fig. 17 is a schematic diagram showing an external side of the front shell half attached thereto with the capsules according to the second preferred embodiment of the present invention;
Fig. 18 is a schematic diagram showing the structure of the capsule according to the second preferred embodiment of the present invention;
Fig. 19 is a schematic diagram of the capsule according to the second preferred embodiment of the present invention, which is provided thereon with a single-sided adhesive film and a heating element;
Fig. 20 is a front view of the structure of Fig. 19; and
Fig. 21 is a cross-sectional view of the structure of Fig. 20 taken along line A-A.

### Description of Reference Numerals in Drawings

1-Shoulder Strap; 2-Defibrillation Pad; 3-Sensing Electrode; 4-Host; 5-Airbag;
100, 100'-Electrode plate; 110-Hermetic Shell; 111-Inflation Port; 112, 112'-Overflow Aperture; 113-Exposed Surface; 114, 114'-Conductive Panel; 115, 115'-Front Shell Half; 1151, 1151'-Mounting Hole; 116, 116'-Rear Shell Half; 117-Cable Port; 118-Protrusion; 119-Screw;
120, 120'-Capsule; 121-Body; 122-Cavity; 123, 123'-Outlet Orifice; 124-Cover; 125-Mounting Recess; 124'-Single-sided Adhesive Film;
130-Heating Element; 141-Inflation Hole; S-Conductive Paste; 200-Vest; 300-Sensing Electrode; 400-Host; 500-Airbag.

### DETAILED DESCRIPTION

Embodiments of the present invention will be described below by way of particular examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, unless the context clearly dictates otherwise. As used herein, the term "multiple" means two or more, unless the context clearly dictates otherwise. As used herein, the term "or" is employed in the sense including "and/or" unless the context clearly dictates otherwise. Additionally, it is to be noted that reference numerals and/or characters may be repeatedly used throughout the embodiments disclosed hereafter. Such repeated use is intended for simplicity and clarity and does not imply any relationship between the discussed embodiments and/or configurations. It is to be also noted that when a component is described herein as being "connected" to another component, it may be connected to the other component either directly or via one or more intervening elements.

In general terms, the present invention seeks to provide an electrode plate primarily for use in cardiac defibrillation. The electrode plate includes a hermetic shell and a capsule housed in the hermetic shell. The hermetic shell has an inflation port and an overflow aperture. The overflow aperture is provided in an exposed surface of the hermetic shell, which is conductive. The capsule includes a body and a cover. The body defines a hollow cavity and an outlet orifice in communication with the cavity. The cavity is configured for storage of a conductive paste therein and is isolated from a hollow internal space of the hermetic shell. The cover is provided over the outlet orifice so as to close the outlet orifice. An opening formed in the cover when it is broken communicates with the outlet orifice and the overflow aperture.

It operates by closing the outlet orifice by the cover when not under load, maintaining the outlet orifice in a closed configuration where the conductive paste is prevented from being released. When the cover is broken open under load, the outlet orifice transitions from the closed configuration to an open configuration where the conductive paste is allowed to be released. In addition, when an external gas is introduced into the hermetic shell via the inflation port, the cover may be heated to break, bringing the outlet orifice into the open configuration. Alternatively, the cover may break open under the action of a pressure of the filled gas, bringing the outlet orifice into the open configuration. Still alternatively, the cover may break as a result of both the above two actions, bringing the outlet orifice into the open configuration. Regardless of the method used, once the cover is broken, an opening created therein will come into communication with the outlet orifice of the capsule and the overflow aperture on the hermetic shell, allowing the conductive paste to be urged by the pressure of the filled gas to flow out of the outlet orifice through the overflow aperture onto the exposed surface. As the exposed surface is configured to be brought into contact with a patient's skin, the conductive paste filled between the patient's skin and the exposed surface can result in an impedance decrease.

It is to be understood that the electrode plate of the present invention is capable of automatic application of the conductive paste, which can help a wearable defibrillation device to provide timely protection to the patient. Moreover, in case of multiple capsules being used, even when one or more of the capsules break first, the breakage of the remaining one(s) will not be affected at all. Therefore, it is ensured that all the capsules can be broken to release a sufficient amount of the conductive paste to enable the defibrillator device to function in a timely way to protect the patient in emergency situations. In particular, compared to causing breakage of the capsule by an explosion of a gas-producing agent, breaking the cover by a heating action and/or under the action of a gas pressure enables control of the gas pressure by an inflation pump included in a wearable defibrillation device, thus ensuring that the cover is broken at a maximum limit pressure lying within an acceptable safe pressure range for the human body and ensuring safety in release of the conductive paste. Further, heating the cover enables the conductive paste that is squeezed out to be also heated and thus have increased fluidity. In this way, release of the conductive paste is enhanced. Further, the hermetic shell of the electrode plate is allowed to be designed sufficiently lightweight and slim to increase wearing comfort and compliance. Meanwhile, since the electrode plate of the present invention is a disposable product, it is inexpensive and can relieve the patient's treatment burden.

The electrode plate and wearable defibrillation device provided in the present invention will be further described below with reference to the accompanying drawings which present several preferred embodiments of the invention.

### Embodiment 1

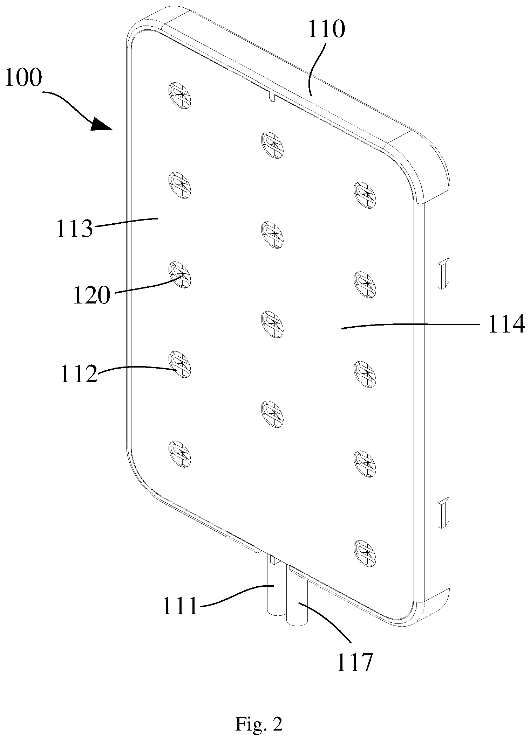

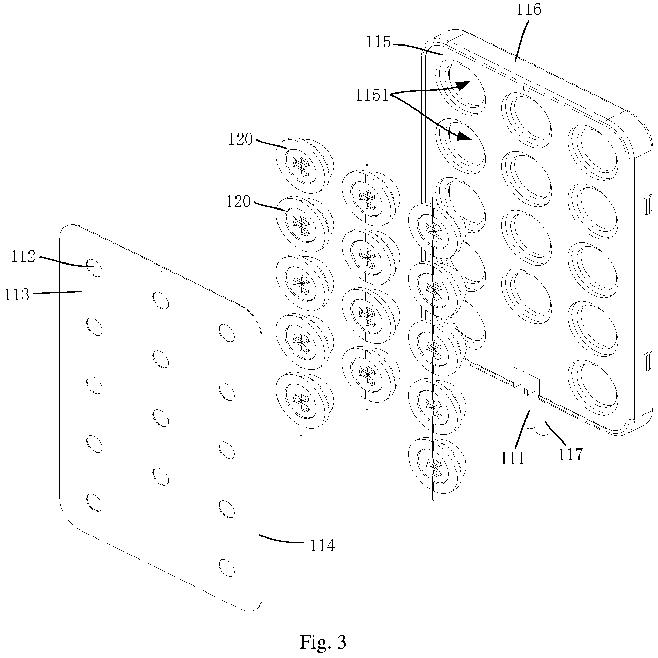

Figs. 2 and 3 are schematic assembled and exploded views of an electrode plate according to a first preferred embodiment of the present invention. As shown in Figs. 2 and 3, the electrode plate 100 in this embodiment is intended for use in cardiac defibrillation and in particular includes a hermetic shell 110 and a capsule 120. The capsule 120 is housed in the hermetic shell 110 and configured for storage of a conductive paste therein.

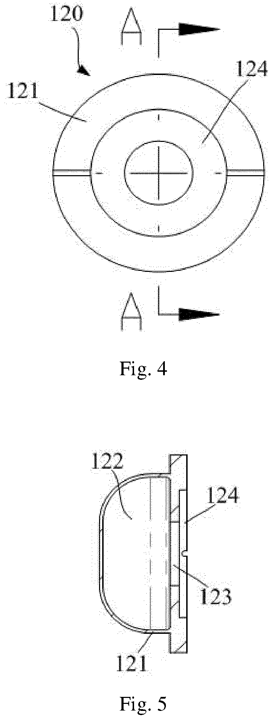

Figs. 4 and 5 are a front view and a cross-sectional view taken along line A-A in Fig. 4 of the capsule in the first preferred embodiment of the present invention, respectively. As shown in Figs. 4 to 5, the capsule 120 includes a body 121, the body 121 defines a hollow cavity 122 in which the conductive paste is stored. The cavity 122 has an outlet orifice 123, the outlet orifice 123 is capable of assuming either an open or closed configuration. In the closed configuration of the outlet orifice 123, the cavity 122 is a closed space, and the conductive paste cannot be released therefrom. In the open configuration of the outlet orifice 123, the cavity 122 is an open space, and the conductive paste is allowed to be released therefrom.

In order to enable the above configurations of the outlet orifice 123, the capsule 120 further includes a cover 124 disposed over the outlet orifice 123, the cover 124 is configured to close the outlet orifice 123 of the cavity 122. In this embodiment, the cover 124 may be either separate from or integral with the body 121, without limiting the present invention in any way. In case of the cover 124 being separate from the body 121, the cover 124 may be glued to the body 121. In practical use, when not under load, the cover 124 is intact and effectively closes the outlet orifice 123, maintaining the outlet orifice 123 in the closed configuration. When the cover 124 is broken under load, the outlet orifice 123 will transition to the open configuration from the closed configuration. It should be noted that, in the context of the present invention, the breakage may include both incomplete and complete separation of two parts which were previously connected to each other. Further, the breakage of the cover 124 may be a result of partial melting of the cover 124 and the creation of a hole therein when it is heated, rupturing or tearing of the cover 124 when a gas pressure in the hermetic shell 110 exceeds a maximum pressure that the cover 124 can withstand, or both.

In greater particularity, in order to enable breakage of the cover 124, in some embodiments, the cover 124 is made of a material with a low melting point, such as an ethylene vinyl acetate (EVA) copolymer with a melting point of 75 °C so that a hole will be created in the cover 124 when it is heated, which communicates with the outlet orifice 123 and thus brings the outlet orifice 123 into the open configuration. In some embodiments, the cover 124 will be ruptured or torn under the action of a pressure of a gas filled in the hermetic shell 110 that acts on the capsule 120, and the tear in the cover 124 communicates with the outlet orifice 123, bringing the outlet orifice 123 into the open configuration. In this case, it is more preferred to further heat the cover 124 to soften it and lower its structural strength so that the cover 124 will be more easily ruptured or torn under the action of the pressure of the gas. Thus, the heating action can make the cover 124 more likely to be broken or even itself create a hole in the cover 124. It is to be understood that, irrespective of whether the breakage is caused by the heating action or by the pressure of the gas, the pressure of the gas filled in the hermetic shell 110 is controllable and much lower than a pressure resulting from an explosion of a gas-producing agent. Therefore, the hermetic shell 110 is allowed to have lower structural strength and can be designed to sufficiently lightweight and slim. Further, heating the cover 124 is also advantageous in that the conductive paste that is squeezed out can be also heated and thus have increased fluidity. As a result, release of the conductive paste is enhanced.

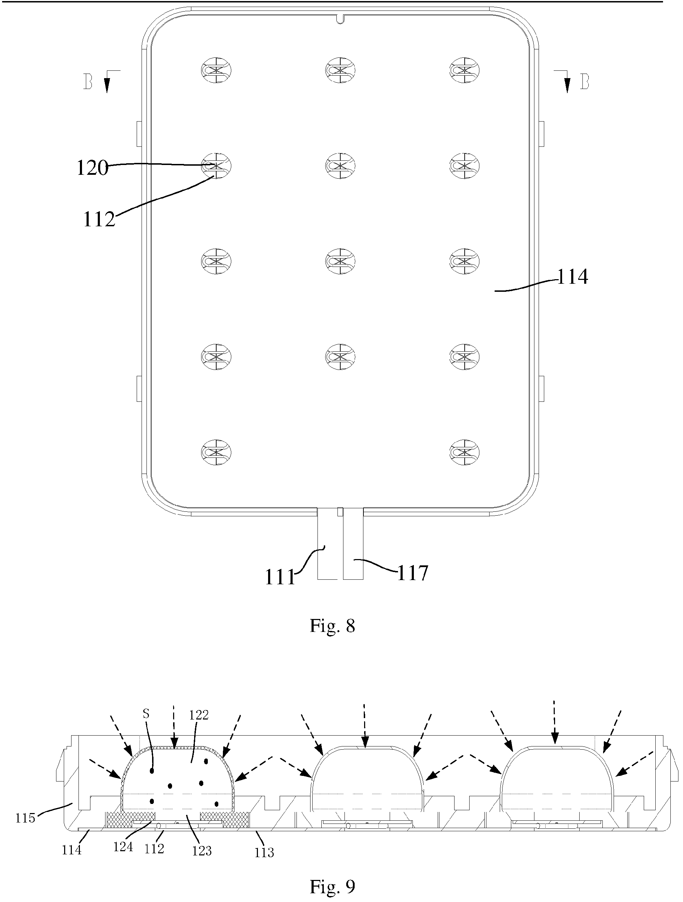

Returning to Figs. 2 and 3, the hermetic shell 110 has an inflation port 111 and an overflow aperture (a paste-overflow aperture) 112. An external gas (preferably, air) can be introduced into the hermetic shell 110 via the inflation port 111. Here, the hermetic shell 110 may be inflated using an inflation pump included in a wearable defibrillation device, and a pressure of the filled gas can be controlled to a value that can cause breakage of the cover 124. Preferably, it is designed that breakage of the cover 124 takes place when the gas pressure lies between 1.5 and 3.0 atmospheric pressures. The overflow aperture 112 is disposed in an exposed surface 113 of the hermetic shell 110 in alignment with the outlet orifice 123 of the capsule 120. Reference is now made to Fig. 9. When the cover 124 is broken and the outlet orifice 123 is thereby brought into the open configuration, the conductive paste S is urged by the pressure of the filled gas to flow out through the outlet orifice 123 and the overflow aperture 112 onto the exposed surface 113. It is to be understood that, during use, the conductive paste S is located in the closest vicinity of the patient's body in order to reduce contact resistance between the electrode plate 100 and the patient's skin. It is also to be understood that the exposed surface 113 is not limited to being made of a conductive metal. Rather, it may also be made of a non-metallic material such as conductive rubber. It would be appreciated that the cavity 122 of the capsule 120 is isolated from a hollow internal space of the hermetic shell 110. In this way, the conductive paste that has flowed out of the capsule 120 through the outlet orifice 123 will not enter the internal space of the hermetic shell 110. Additionally, breakage of the cover 124 will not lead to a pressure drop within the hermetic shell 110 and thus ensure hermeticity of the hermetic shell 110.

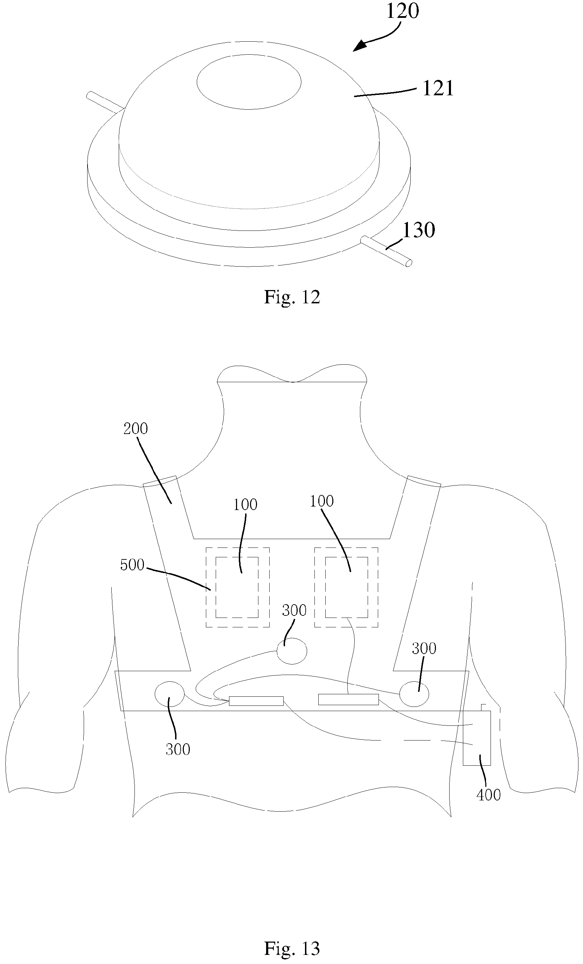

As shown in Fig. 13, in this embodiment, there is also provided a wearable cardioverter-defibrillator (WCD) device including a vest 200 and, provided on the vest 200, an electrode plate 100, sensing electrodes 300, a host 400 and an airbag 500. With reference to Fig. 13, in practical use, after the vest 200 is worn on the patient, the WCD device is able to effect cardiac defibrillation. The WCD device operates by sensing an electrical signal from the heart and feeding the signal to the host 400 by the sensing electrodes 300. The host 400 responsively produces an electrocardiogram and makes a determination based thereon. If the host 400 determines that the patient is in need of defibrillation, the airbag 500 and the electrode plate 100 are inflated. The inflated airbag 500 compresses the electrode plate 100 against the patient's skin, and the inflation of the electrode plate 100 cause expansive deformation of a hermetic shell 110 thereof. Accordingly, capsules 120 within the hermetic shell 110 will also deform under the pressure of the gas. When the pressure rises to a predetermined value, outlet orifices 123 of the capsules 120 will be broken open, and a conductive paste S in the capsules 120 will be urged by the gas pressure to flow out through overflow apertures 112 on the hermetic shell 110 into a gap between an exposed surface 113 and the patient's skin, resulting in an impedance drop. After that, the electrode plate 100 delivers a high DC voltage for defibrillation treatment.

It is to be understood that the electrode plate 100 and the airbag 500 may be inflated simultaneously or successively, without limiting the present invention in any away, with simultaneous inflation being more preferred. It is also to be understood that structural strength of the hermetic shell 110 of the present invention should suffice to prevent the electrode plate 100 from experiencing defibrillation performance degradations due to its deformations that occur when it is compressed or twisted during daily physical activities of the patient, prevent the capsules 120 therein from being accidentally compressed and broken, and prevent breakage of the shell itself during the inflation at a gas pressure below a predetermined value. At the same time, the hermetic shell 110 should also have a certain degree of flexibility which ensures wearing comfort of the patient. For these reasons, the hermetic shell 110 is configured with both sufficient support strength and a certain degree of flexibility. Apart from this, the WCD device of the present invention operates automatically to effect timely and satisfactory protection of the patient. Further, before the electric defibrillation by the electrode plate 100, the conductive paste is automatically coated to effectively reduce contact resistance between the electrode plate 100 and the skin, ensuring that the defibrillation is conducted in a safe and effective style. Furthermore, the electrode plate 100 is a low-cost disposable product, and its use can significantly relieve the patient's burden for the surgery.

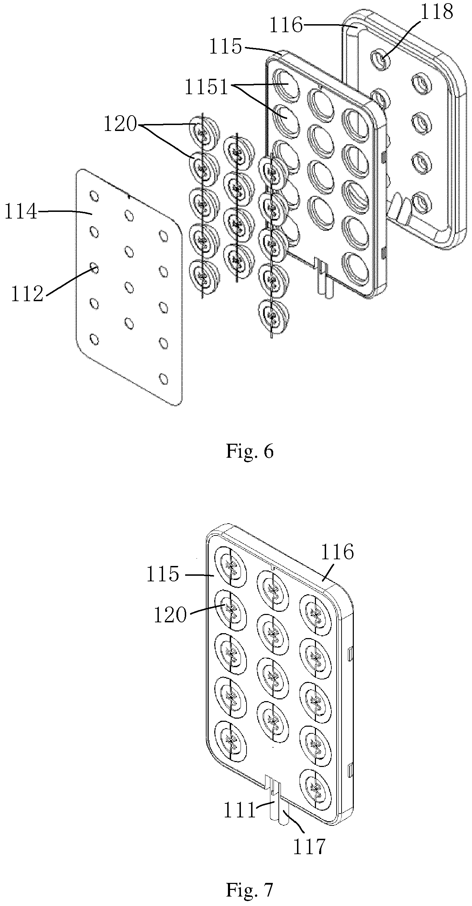

Reference is additionally made to Figs. 6 and 7, in conjunction with Figs. 2 and 3. For ease of understanding, a conductive panel 114 is omitted in Fig. 7. The hermetic shell 110 may include the conductive panel 114, a front shell half 115 and a rear shell half 116. The front shell half 115 and the rear shell half 116 are both insulators. The front shell half 115 and the rear shell half 116 are coupled together to form the hermetic shell, and the capsules 120 are provided on the front shell half 115. The exposed surface 113 is provided by the conductive panel 114, which is placed on the front shell half 115 on the side thereof away from the rear shell half 116. The overflow apertures 112 are provided in the conductive panel 114 in such a manner that each of the overflow apertures 112 is aligned with a respective one of the outlet orifices 123. In this embodiment, multiple (i.e., at least two) capsules 120, and thus the same number of overflow apertures 112, may be included. The number of the capsules 120 may depend on an actual defibrillation area. For example, in this embodiment, three sensing electrodes 300 (see Fig. 13) may be included, corresponding to a defibrillation area not smaller than 60 cm². In this case, fourteen or fifteen capsules 120 may be arranged in the hermetic shell 110 in order to accommodate a greater amount of the conductive paste. These capsules 120 are usually uniformly scattered in order to achieve high space utilization. The uniform scattering is preferably in the form of a linear array. More preferably, the capsules 120 are equally sized.

Additionally, the front shell half 115 has higher material strength than the rear shell half 116. In this way, the front shell half 115 can impart sufficient support strength to the electrode plate 100, which protects the electrode plate 100 against compressive deformations that may occur during physical activities of the patient (e.g., turning over during sleep, etc.) and prevents the capsules 120 from being damaged by accident. At the same time, the rear shell half 116 can impart sufficient flexibility to the electrode plate 100, which enables its easy expensive deformation during the inflation and provides increased wearing comfort. The front shell half 115 is preferably made of non-metallic material, which is more lightweight than a metallic material and can thus additionally increase wearing comfort. Examples of the non-metallic material of which the front shell half 115 is made may include, but are not limited to plastics and other materials having suitable hardness and strength characteristics. The rear shell half 116 is generally made of a relatively pliable non-metallic material with a certain degree of crush resistance, such as silica gel, latex, TPU or PVC. It is to be understood that in other embodiments, the material strength of the front shell half 116 may be equal to that of the rear shell half 116. The conductive panel 114 is made of a conductive metallic or non-metallic material, the conductive panel 114 has an external surface which serves as the aforementioned exposed surface 113. In this embodiment, the conductive panel 114 is a metal plate, which can easily compress and hold the capsules 120 to prevent the dislodgement of the capsules 120 during the inflation process. During assembly, the metal plate may be adhesively bonded to the front shell half 115 by means of a glue applied to peripheral edges of the metal plate and a surface thereof proximate the front shell half 115. This entails a simple process that facilitates assembly. Specifically, the front shell half 115 may be provided therein with the mounting through holes 1151, the number of the mounting holes 1151 is the same as the number of the capsules 120. In practice, after the front shell half 115 and the rear shell half 116 are coupled together, the capsules 120 may be placed into the mounting holes 1151 in the front shell half 115, followed by the attachment of the conductive panel 114. In this embodiment, the coupling between the front shell half 115 and the rear shell half 116 may be accomplished by gluing, threading or snapping. The present invention is not limited to any particular coupling method for the front shell half 115 and the rear shell half 116. The inflation port 111 is provided on the hermetic shell.

Figs. 8 and 9 are a front view and a cross-sectional view taken along line B-B in Fig. 8 of the electrode plate in the first preferred embodiment of the present invention, respectively. In more particularity, the structure shown in Figs. 8 and 9 can be obtained after the attachment of the conductive panel 114, in which each of the capsules 120 is received in a respective one of the mounting holes 1151 in the front shell half 115, and the conductive panel 114 compressively covers the front shell half 115. Each capsule 120 is partially located within the hermetic shell formed as a result of the coupling of the front shell half 115 and the rear shell half 116. When inflated, a gas pressure will act on each capsule 120 in a manner as indicated by the arrows in the figure and cause the capsule 120 to deform. As a result, a pressure in an internal cavity 122 of the capsule 120 will rise, and when the pressure increases to a level exceeding a maximum pressure that a cover 124 can withstand, the cover 124 will be ruptured or torn, opening the outlet orifice 123. Further, the conductive paste S will be urged by the gas pressure to flow out of the outlet orifice 123 through the overflow aperture 112 onto the exposed surface 113. Here, the outlet orifice 123 is not limited to being opened by the gas pressure, because it may also be opened by heating the cover 124 during the inflation process to soften and melt it, or even thus create a hole in it.

Further, the present invention is not limited to any particular material of which the capsule 120 is made, and examples of the material may include, but are not limited to, silica gel, latex, TPU, etc. Preferably, multiple such capsules 120 are used. In fact, for a given defibrillation pad, as long as its internal space allows, as many as possible capsules 120 may be arranged therein, in order to attain an increased amount of released conductive paste, a larger coated area and higher defibrillation safety. It is also to be understood that, when multiple capsules 120 are used, as shown in Fig. 9, all the capsules 120 are subjected to the same gas pressure condition. Even when the capsules 120 are not simultaneously broken open, one or more of them that are broken open before the remaining one(s) will not lead to a pressure drop within the hermetic shell 110, which makes the remaining capsule(s) 120 impossible to be broken any longer. Therefore, it can be ensured that all the capsules 120 will be broken open to release a sufficient amount of the conductive paste. Such that the defibrillator device can function in a timely way to protect the patient, thereby ensuring the reliability of the release of the conductive paste.

Referring back to Figs. 2 and 3, the hermetic shell 110 further includes a cable port 117 for passage therethrough into the hermetic shell 110 of a cable for providing an electric current to the conductive panel 114 and supplying power to the heating elements 130 described below. Referring again to Fig. 6, protrusions 118 are preferably on the rear shell half 116 on the side thereof facing the front shell half 115. The protrusions 118 are configured for abutment of the capsules 120 thereagainst. During daily wearing, the protrusions 118 can buffer external forces which may bring damage to the capsules 120 and thereby prevent accidental breakage of them. The present invention is not limited to any particular shape of the protrusions 118. Preferably, the protrusions 118 are integrally formed with the rear shell half 116. The present invention is also not limited to any particular shape of the hermetic shell 110, and possible shapes may include, but are not limited to, the rectangular parallelepiped shown in the figures. In general terms, designing the hermetic shell 110 as a rectangular parallelepiped is advantageous in easy manufacturing, a large accommodating space for the capsules and hence high space utilization.

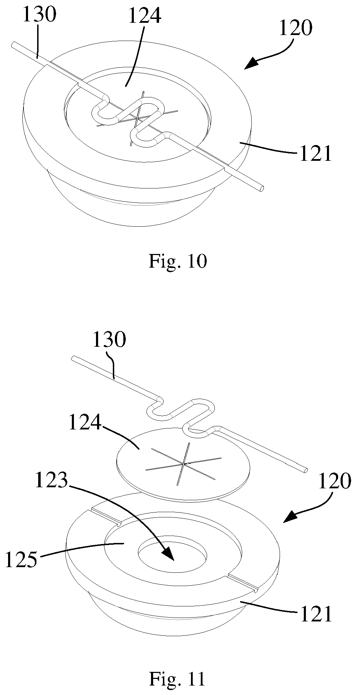

Fig. 10 is a schematic front assembled view of one capsule in the first preferred embodiment of the present invention. Fig. 11 is a schematic exploded view of the capsule in the first preferred embodiment of the present invention. Fig. 12 is a schematic back assembled view of one capsule in the first preferred embodiment of the present invention. As shown in Figs. 10 to 12, in this embodiment, the body 121 and the cover 124 in the capsule 120 are fabricated separately. The body 121 defining the hollow cavity 122 is a thin-walled part which may be made of a pliable material with high toughness, such as silica gel, latex, PVC or the like. The outlet orifice 123 is defined at one end of the cavity 122, and a concave mounting recess 125 is defined around the outlet orifice 123. The cover 124 fits in the mounting recess 125 of the body 121. The cover 124 is configured as a sheet-like structure and may have, without limitation, a circular shape. In some embodiments, the cover 124 is made of composite paper and provided thereon with a non-through indentation serving as a weakened feature. It this way, it is ensured that the cover 124 will break open first at the weakened feature under a given gas pressure. There may be one or more (e.g., two, three or more) such weakened features, and they are not limited to assuming the form of indentations. Instead, they may be internal cavities or other weakened features formed on the cover 124 by a local heat treatment. Additionally, the weakened features may be a combination of various structures. It is to be understood that a maximum limit structure for the weakened features is lower than that of the rest of the cover 124, ensuring that the cover 124 breaks first at one or more of the weakened features.

Further, the electrode plate 100 may further include heating elements 130 (see Fig. 10) attached to the respective covers 124, each heating element 130 and the corresponding outlet orifice 123 are arranged on opposing sides of the corresponding cover 124. When energized, the heating elements 130 will heat and thus melt the covers 124. It is to be understood that, as used herein, the term "melt" is meant to include softening the material until breakage takes place or not. The present invention is not limited to any particular structure of the heating elements 130. For example, they may be electrical heating wires or inductance coils, with electrical heating wires being more preferred because they are simple in structure and can facilitate assembly. The heating elements 130 may be attached to the covers 124 using an adhesive so as to at least partially traverse over the weakened features in order to strengthen the covers 124 around the weakened features to prevent undesired breakage of the covers 124 at the weakened features. Preferably, the adhesive may be formed of a material with a low melting point, which will be molten when the heating elements 130 are energized and therefore will not affect the breakage of the covers 124. In this embodiment, examples of the material with a low melting point of which the adhesive is made may include, but are not limited to, paraffin. The bodies 121 of the covers 124 may be provided with grooves (not labeled) for securing the heating elements 130. For example, as shown in Fig. 10, in case of the heating elements 130 being implemented as electrical heating wires, opposite end portions of the electrical heating wires may be received in the grooves. This can not only facilitate placement of the electrical heating wires, but can also prevent their displacement during use.

### Embodiment 2

The inventors have found from further research that, for a given defibrillation area, the electrode plate will have a smaller space for accommodating the conductive paste when it includes more capsules. Therefore, in fact, a greater number of capsules do not necessarily correspond to an increased amount of the conductive paste that can be loaded. Specifically, in the first embodiment, the electrode plate 100 includes, for example, fourteen capsules 120, which are scattered with gaps being present between them. No matter how large these gaps are, they are totally useless. Relatively speaking, the more such gaps, the smaller an area of the hermetic shell will be left for accommodating the conductive paste. Therefore, the total loadable amount of the conductive paste is subpar. If the electrode plate is thickened to increase the loadable amount of the conductive paste, apart from a cost increase, in particular, the electrode plate will become bulkier and heavier, possibly causing discomfort to the patient. Moreover, a greater number of capsules require the use of more heating elements, which will not only lead to greater power consumption but also necessitates the use of a more powerful battery that is typically heavier and detrimental to the overall wearing comfort of the wearable device. For these reasons, in the second embodiment, with the defibrillation area being maintained, fewer but longer and wider capsules are included in an electrode plate with a reduced thickness. In this way, the electrode plate has a larger space capable of accommodating more conductive paste and can be designed to be even more lightweight and slimmer to impart more wearing comfort to the patient. Additionally, a smaller number of heating elements are allowed to be used, resulting in a decrease in power consumption. Further, this power-saving design allows the use of a less powerful and thus lighter battery, further enhancing the device's wearing comfort.

It would be appreciated that the electrode plate of the second embodiment is essentially the same in structure as that of the first embodiment. Identical features will not be explained in detail again. That is, the various variants of the first embodiment are also applicable to the second embodiment. Thus, the following description focuses only on their differences.

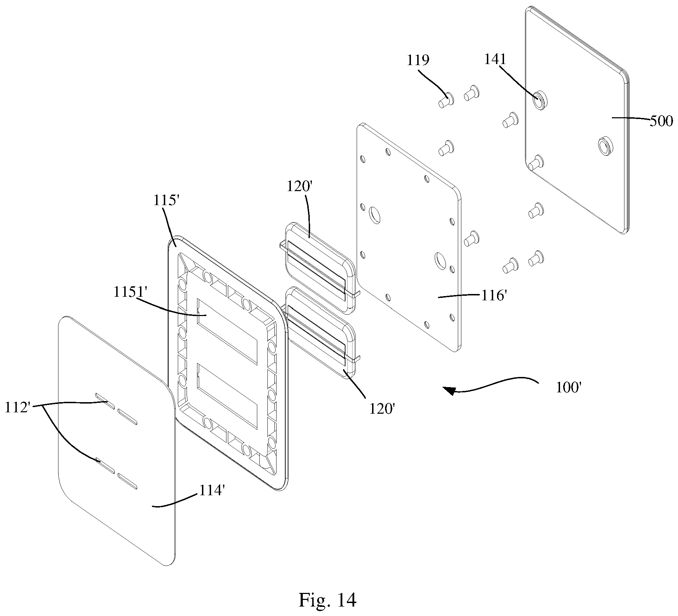

Fig. 14 is a schematic exploded view of the electrode plate according to the second preferred embodiment of the present invention. As shown in Fig. 14, the electrode plate 100' according to the second embodiment incorporates only two capsules 120'. In comparison with the first embodiment, the electrode plate 100' has a larger effective available space, and each capsule 120' has a larger size. Thus, for a given area, the electrode plate 100' according to the second embodiment can accommodate a greater amount of the conductive paste and is more lightweight and slimmer, imparting more wearing comfort to the patient. In the second embodiment, it is preferred that two capsules 120' are included to enable the largest possible effective available space and a significantly increased amount of the conductive paste that can be loaded. The present invention is not limited to any particular shape of the capsules 120' and the capsules 120 in the second and first embodiments. For example, the capsules 120 in the first embodiment are not limited to having a semispherical shape, and the capsules 120' in the second embodiment are not limited to having a rectangular shape.

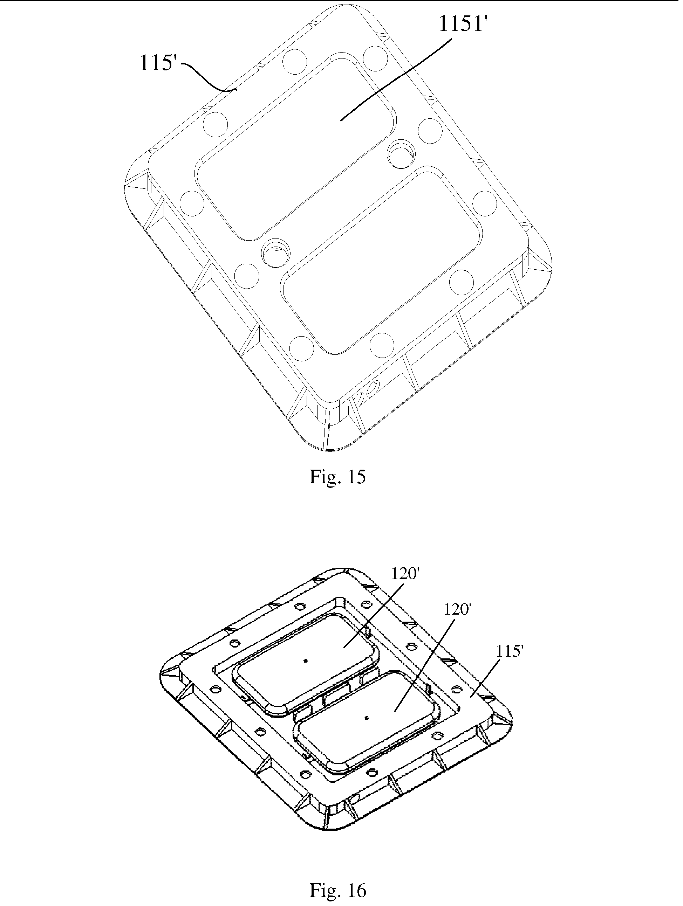

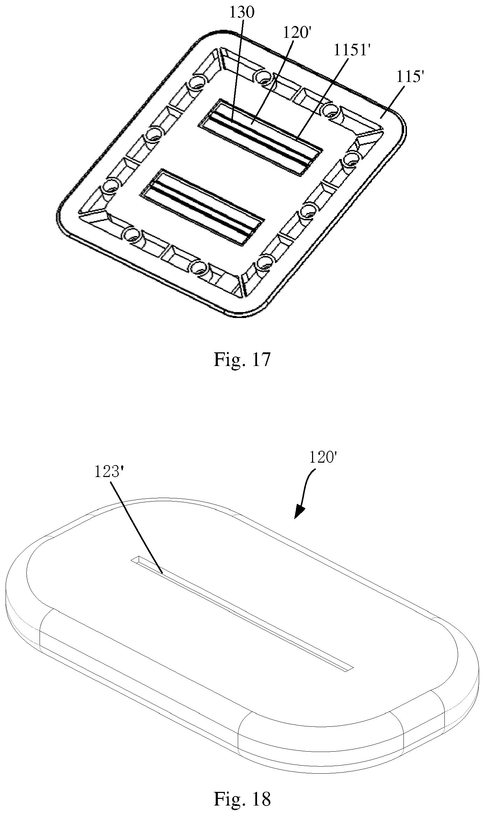

As shown in Figs. 14 to 17, in the second embodiment, it is preferred to provide two mounting holes 1151' in a front shell half 115'. The mounting holes 1151' generally match the capsules 120' in terms of shape and size. In addition, the capsules 120' are sealingly attached to the respective mounting holes 1151' by an adhesive applied to sidewalls of the mounting holes 1151' in circumferential. This not only entails a simple process that facilitates assembly but also avoids impeding the placement of heating elements 130, thereby allowing the electrode plate 100' to have an even smaller thickness. As shown in Fig. 18, in order to ensure uniform application of the conductive paste onto a conductive panel 114', each capsule 120' is designed to have a larger elongate outlet orifice 123'. Additionally, as shown in Fig. 14, each outlet orifice 123' is provided with multiple overflow apertures 112' arranged in a row. Each overflow aperture 112' is preferred to be also elongate and have the same lengthwise direction as the corresponding outlet orifice 123'. More specifically, each outlet orifice 123' may be provided with two overflow apertures 112' that are arranged in a row. Providing each outlet orifice 123' with multiple overflow apertures 112' in a row is advantageous not only in strengthening the conductive panel 114' and thereby preventing its deformation that may affect the assembly process but also in facilitating squeezing of the conductive paste from the multiple overflow apertures 112', which is conducive to its uniform application.

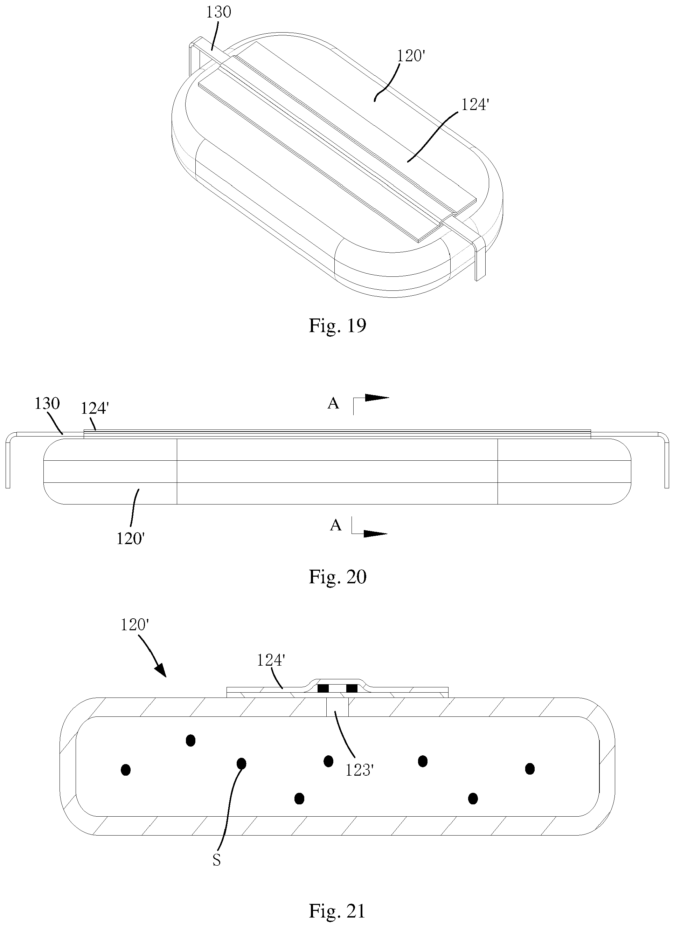

As shown in Figs. 19 to 21, each capsule 120' is also provided with a cover to close the outlet orifice 123'. However, in the second embodiment, the cover is bonded with a single-sided adhesive film 124' (which is a film with an adhesive applied to only one side thereof). The single-sided adhesive film 124' is attached on one side thereof over the outlet orifice 123' so as to close the outlet orifice 123'. Additionally, a heating element 130 is secured by the single-sided adhesive film 124' and is connected to a host 400 via a lead. When necessary, the heating element 130 is energized to heat the single-sided adhesive film 124' and burn a hole through the single-sided adhesive film 124', opening the outlet orifice 123'. Further, in order to prevent insufficient contact between the heating element 130 and the single-sided adhesive film 124', the heating element 130 is preferably to be inserted through the single-sided adhesive film 124'. In practice, the single-sided adhesive film 124' may consist of two layers, and the heating element 130 may be inserted between the layers of the single-sided adhesive film 124'. The single-sided adhesive film 124' may be formed of a material with a low melting point, such as composite paper or tinfoil. Alternatively, it may also be made of EVA, PE or another material.

Referring back to Fig. 14, in the second embodiment, a rear shell half 116' is fastened to the front shell half 115' by several screws 119. In addition, in the second embodiment, the hermetic shell is provided with inflation holes 141 in communication with an airbag 500. Specifically, the inflation holes 141 are attached to the rear shell half 116' and extend into the hermetic shell 110, allowing shared use of a common gas source by the airbag 500 and the hermetic shell 110. The airbag 500 may be made of a flexible material such as silica gel, latex or polyurethane. When inflated, the airbag expands and compresses the conductive panel 114' against the patient's skin.

The electrode plate 100' of this this embodiment operates in the same way as that of the first embodiment. When the host 400 determines that the patient is in need of defibrillation, the electrode plate 100' is inflated, and the airbag 500 expands to compress the electrode plate 100' against the patient's skin. At the same time, as the capsules 120' are also subject to a certain pressure, the heating elements 130 are energized several seconds after the beginning of the inflation. As a result, holes are burnt in the single-sided adhesive films 124', and the conductive paste is released from the holes. Under the action of the gas pressure, the conductive paste flows through the overflow apertures 112' onto the patient's skin, reducing electrical resistance between the electrode plate and the patient's skin.

In summary, this application provides an electrode plate, which allows an inflation pump included in a WCD device to be used to introduce a gas into a hermetic shell via an inflation port to cause expansive deformation of the hermetic shell. In this process, when a cover on a capsule is broken, under the action of a gas pressure within the hermetic shell, a conductive paste within the capsule can flow out of an opening in the cover resulting from the breakage through an overflow aperture onto a conductive exposed surface. In this way, apart from automatic application of the conductive paste which enables timely protection of a patient, higher safety can be obtained by avoiding the use of a gas-producing agent for creating a gas pressure rise by explosively producing a gas.

In addition, instead of a sufficiently high pressure resulting from an explosion of a gas-producing agent, the breakage of the cover on the capsule can be simply accomplished by introducing an appropriate amount of gas into the hermetic shell. In this process, the cover may break as a result of a pressure of the gas introduced into the hermetic shell, a heating action, or both. In these approaches for causing the breakage, the gas pressure can be controlled by the inflation pump included in the WCD device, thus ensuring that the cover is broken at a maximum limit pressure lying within an acceptable safe pressure range for the human body and ensuring safety in release of the conductive paste. In particular, unlike a gas pressure produced by an instantaneous explosion of a gas-producing agent, the pressure of the gas filled in the hermetic shell is balanced, ensuring simultaneous breakage of all capsules and hence release of a sufficient amount of the conductive paste. As such, the defibrillator device is enabled to function in a timely manner to protect the patient in emergency situations.

Apart from these, heating the cover enables the conductive paste that is squeezed out to be also heated and thus have increased fluidity. In this way, release of the conductive paste is enhanced. In particular, breaking the cover by a heating action and/or under the action of a gas pressure created by an inflation action enables the hermetic shell of the electrode plate to be designed sufficiently lightweight and slim to increase wearing comfort and compliance. Moreover, the electrode plate of the present invention is an inexpensive disposable product and can relieve the patient's treatment burden.

Further, in order to ensure breakage of the cover under the action of the gas pressure, the cover is preferably provided thereon with a weakened feature preferably in the form of a non-through indentation. In this case, when the cover is subjected to a thermal load and/or a pressure load, the weakened feature will be ruptured first. This is advantageous in structural simplicity, ease of operation and safe and reliable breakage of the cover. Additionally, in order to ensure that the capsule will not be accidentally damaged during daily wearing, an adhesive is preferably applied around the weakened feature to enhance its structural strength. Under normal circumstances, the adhesive may be heated and molten by the heating element to ensure breakage of the cover.

Furthermore, according to embodiments of the present invention, in addition to sufficient structural strength, the hermetic shell of the electrode plate can desirably avoid the electrode plate from being twisted and deformed due to the patient's physical activities during his/her daily wearing, which may lead to defibrillation performance degradations of the electrode plate. Moreover, after inflation, it can expand and deform with exhibiting a certain degree of flexibility, additionally enhancing wearing comfort. In particular, multiple capsules may be included to enable an increased amount of released conductive paste, a larger coated area and higher defibrillation safety. Additionally, since all the capsules are subject to the same gas pressure conditions, even if the capsules fails to be simultaneously broken, one or more of them that have been broken prior to the other(s) will not lead to a pressure drop in the hermetic shell and thus will not make the remaining one(s) impossible to be broken any longer. Therefore, reliable release of the conductive paste can be ensured. Further, according to this application, a greater amount of the conductive paste can be loaded in a smaller number of capsules for a given defibrillation area. In this way, an increased amount of the conductive paste can be released to achieve even safer defibrillation. In particular, the electrode plate can be made even more lightweight and slimmer, imparting even better wearing comfort to the patient.

It is to be understood that features of the present invention have been disclosed in the foregoing preferred embodiments to provide a better understanding of the invention to those skilled in the art. It would be appreciated by those skilled in the art that, on the basis of the disclosure herein, it would be easy to modify the present invention while still achieving the same objects and/or advantages as the embodiments disclosed herein. Those skilled in the art would also recognize that such similar configurations do not depart from the scope of disclosure of this invention and could be subject to various changes, substitutions, and alterations without departing the scope of disclosure of the invention.

## Claims

1. An electrode plate for use in cardiac defibrillation, the electrode plate comprising a hermetic shell and a capsule disposed in the hermetic shell, the hermetic shell having an inflation port and an overflow aperture, the overflow aperture provided in an exposed surface of the hermetic shell, wherein the exposed surface has electrical conductivity,
the capsule comprising a body and a cover, the body defining a hollow cavity and an outlet orifice in communication with the cavity, the cavity configured for storage of a conductive paste therein, the cavity isolated from a hollow internal space of the hermetic shell, the cover disposed over the outlet orifice and configured to close the outlet orifice, the cover when broken open having an opening therein resulting from the breakage, which comes into communication with the outlet orifice and the overflow aperture.

2. The electrode plate according to claim 1, wherein the cover is configured to be broken open when a gas is introduced into the hermetic shell by heating and/or under the action of a pressure of the gas.

3. The electrode plate according to claim 2, further comprising a heating element attached to the cover, wherein the heating element and the outlet orifice are on opposing sides of the cover, the cover is configured to be heated, molten and broken open when the heating element is energized.

4. The electrode plate according to claim 1 or 2, wherein the cover is configured with a weakened feature which is able to withstand a maximum pressure lower than a maximum pressure that the rest of the cover is able to withstand.

5. The electrode plate according to claim 4, further comprising a heating element, wherein the heating element and the outlet orifice are on opposing sides of the cover, the heating element attached to the cover by an adhesive, at least part of the adhesive is applied to the weakened feature, and the adhesive is configured to be heated and molten when the heating element is energized, wherein the cover is further configured to be heated, molten and broken open when the heating element is energized.

6. The electrode plate according to claim 4, wherein the weakened feature is configured as a non-through indentation.

7. The electrode plate according to claim 1, wherein the body defines a mounting recess around the outlet orifice, wherein the cover is sheet-shaped and fits into the mounting recess of the body.

8. The electrode plate according to claim 3, wherein the cover is a single-sided adhesive film consisting of two layers, the heating element being inserted between the two layers.

9. The electrode plate according to claim 1 or 2, comprising a plurality of the capsules, each of the capsules defining an elongate outlet orifice provided with a plurality of overflow apertures arranged in a row, each of the overflow apertures being elongate and having a same lengthwise direction as the outlet orifice.

10. The electrode plate according to claim 1 or 2, wherein the hermetic shell comprises a front shell half and a rear shell half, wherein the front shell half and the rear shell half are both insulators and are coupled together to form the hermetic shell, the front shell half providing the exposed surface, the front shell half having material strength higher than material strength of the rear shell half, the rear shell half configured to expansively deform as a result of inflating the hermetic shell.

11. The electrode plate according to claim 10, wherein the rear shell half is provided with a protrusion on the side thereof facing the front shell half, the protrusion configured to abut against the capsule.

12. The electrode plate according to claim 1, wherein the outlet orifice and the overflow aperture are configured so that, after the cover is broken open, the conductive paste stored in the cavity flows out of the outlet orifice through the overflow aperture onto the exposed surface.

13. A wearable defibrillation device, comprising a vest, and provided on the vest, a sensing electrode, a host and an airbag, the wearable defibrillation device further comprising the electrode plate as defined in any one of claims 1 to 12, which is provided on the vest.
